# EUROPÄISCHE PATENTSCHRIFT

(11) **EP 3 340 250 B1**
(45) Veröffentlichungstag und Bekanntmachung des Hinweises auf die Patenterteilung: **05.08.2020**
(21) Anmeldenummer: 17197958.6
(22) Anmeldetag: 24.10.2017
(51) Int. Cl.: G16H 40/40

(54) **BAUTEILIDENTIFIKATION BEI DER FEHLERBEHANDLUNG VON MEDIZINISCHEN GERÄTEN**
IDENTIFICATION OF COMPONENTS IN THE ERROR HANDLING OF MEDICAL DEVICES
L'IDENTIFICATION DES COMPOSANTS DANS LE TRAITEMENT DES ERREURS DES DISPOSITIFS MÉDICAUX

(43) Veröffentlichungstag der Anmeldung: 27.06.2018
(73) Patentinhaber: Siemens Healthcare GmbH, 91052 Erlangen (DE)
(72) Erfinder: Görtler, Georg, 91083 Baiersdorf (DE)

(56) Entgegenhaltungen:
- US-A1- 2004 193 958
- US-A1- 2009 125 757

## Beschreibung

Die vorliegende Erfindung betrifft ein Verfahren und ein Datenbanksystem, eine Berechnungseinheit und ein System sowie ein Computerprogrammprodukt zur Identifikation von fehlerhaften, auszutauschenden Bauteilen in einem medizinischen Gerät, das in einem Verbund von Geräten betrieben werden kann.

Ein längerer Ausfall eines medizinischen Gerätes ist im klinischen Betrieb häufig nicht akzeptabel und von daher zeitkritisch. Die Zeitphase, in der ein Ersatzteil gesucht und das fehlerhafte Bauteil durch ein Ersatzteil ersetzt werden muss, sollte möglichst gering sein, um die Ausfallzeit des Gerätes möglichst zu minimieren.

Im Stand der Technik ist es bekannt, dazu computergestützte Verfahren anzuwenden, um den Service von Medizingeräten zu verbessern. Diese Verfahren basieren mitunter auf der Menge der verfügbaren Daten, um daraus Aussagen ableiten zu können. Wenn jedoch nur wenig Daten verfügbar sind, z.B. bei einem neuen Produkt, unmittelbar nach Produktfreigabe, kann es bei diesen Verfahren zu Problemen kommen, weil noch keine oder zu wenig Betriebsdaten vorliegen.

So zeigt die US 2004/0193958 A1 ein System zum Erzeugen eines Service Modells im Rahmen einer Serviceüberwachung von medizinischen Geräten. Dabei werden auch fehlerhafte Bauteile identifiziert.

Ferner zeigt die US 2009/0125757 A1 ein System zur Benutzerführung bei Problembehandlungen von Geräten, darunter auch medizintechnischen Geräten. Der Anwender wird bei der Geräteüberwachung und Problembehandlung Menü-gesteuert geleitet. Ein Fehler wird identifiziert.

Ausgehend von diesem Stand der Technik liegt der vorliegenden Erfindung die Aufgabe zugrunde, die Datenbasis zu erweitern, um im Fehlerfall bereits frühzeitig im Produktzyklus durch ein automatisches Verfahren zu ermitteln, welches Bauteil in einem komplexen medizinischen Gerät ausgetauscht werden soll. Insgesamt sollen die Betriebszeiten der Geräte und die Fehlerbehandlung im Fehlerfall verbessert werden. Insbesondere soll die Suche nach einem adäquaten Ersatzteil beschleunigt werden.

Diese Aufgabe wird jeweils durch einen Gegenstand nach den unabhängigen Ansprüchen gelöst. Vorteilhafte Ausführungsformen sind Gegenstand der abhängigen Ansprüche, der Beschreibung und der Zeichnungen.

Gemäß einem ersten Aspekt wird die Aufgabe durch ein Verfahren zur Identifikation von auszutauschenden (da fehlerhaften) Bauteilen eines medizinischen Gerätes gelöst, das jeweils eine Vielzahl von (möglicherweise unterschiedlichen) Bauteilen umfasst. Bei dem medizinischen Gerät kann es sich um ein MR-Gerät handeln. Das Verfahren umfasst folgende Verfahrensschritte:
- Einlesen von einer Fehlermeldung des Geräts;
- Ausführen eines Identifikationsalgorithmus zur Berechnung und Ausgabe eines Austauschdatensatzes für die eingelesene Fehlermeldung, wobei der Austauschdatensatz ein auszutauschendes Bauteil identifiziert. Der Identifikationsalgorithmus greift dazu mit der eingelesenen Fehlermeldung auf ein Datenbanksystem zu, in dem Fehlermuster mit Austauschdatensätzen abgelegt sind, die aus einem Simulationsmodell berechnet wurden, um als Ergebnis denjenigen Austauschdatensatz zu berechnen, der jeweils der eingelesenen Fehlermeldung zugeordnet ist, wobei in dem Datenbanksystem (DB) Fehlermuster (fmu) gespeichert sind, die automatisch mittels einer Fehlermustererzeugungseinheit (FME) erzeugt werden, wobei die Fehlermustererzeugungseinheit (FME) zur Ausführung eines maschinellen Lernverfahrens auf Basis eines Simulationsmodells ausgebildet ist, das zum kontinuierlichen Lernen und Speichern von Fehlermustern (fmu) durch Korrelation von Eingangsgrößen (e) dient, wobei jeweils ein Fehlermuster (fmu) eine Zuordnung umfasst zwischen einem Fehlertyp oder einer Kombination aus Fehlertypen und einem Austauschdatensatz (ad), der ein auszutauschendes Bauteil (B) indiziert und bei dem das Datenbanksystem (DB) oder die Fehlermustererzeugungseinheit (FME) zum Erzeugen der Fehlermuster (fmu) einen Prozessor (P) umfasst oder mit diesem in Datenaustausch steht, wobei der Prozessor (P) zur Erzeugung der Fehlermuster (fmu) zur Anwendung von Markow-Prozessen ausgebildet ist.

Im Kern hebt die Erfindung darauf ab, die Datenbasis zur Speicherung von Fehlermustern durch den Einsatz von maschinellen Methoden und Algorithmen kontinuierlich zu erweitern, um das Ergebnis für einen erfassten Fehlerfall mit einer optimalen Qualität frühzeitig im Produktlebenszyklus des Gerätes und insbesondere bereits unmittelbar nach Freigabe des Gerätes bereitstellen zu können. Das Ergebnis soll auf Basis von Referenzdaten ein Austauschbauteil identifizieren. Die Referenzdaten umfassen Testdaten aus einem Testbetrieb und Simulationsdaten aus einem Simulationsmodell. Das Simulationsmodell wird während des Testbetriebs aber auch während des realen Gerätebetriebs im Feld laufend erweitert.

In einer vorteilhaften Ausführungsform des Verfahrens wird das Ausführen des Identifikationsalgorithmus durch das Einlesen der Fehlermeldung getriggert wird. Das Verfahren kann somit als proaktives Verfahren betrieben werden. Dies hat den Vorteil, dass das Ergebnis automatisch und schneller bereitgestellt werden kann, da nicht erst eine manuelle Eingabe oder Anfrage abgewartet werden muss.

Vorteilhafterweise kann das Verfahren sogar vollständig ohne Betriebsdaten und unmittelbar nach erstmaliger Betriebsaufnahme bzw. Produktfreigabe des medizinischen Gerätes ausgeführt werden. Dies wird möglich, indem ein Simulationsmodell erzeugt und angewendet wird, das zusätzlich zu den Testdaten noch Simulationsdaten bereitstellt, die als Eingangsgrößen zur Erzeugung von Fehlermustern verwendet werden. Dies hat den Vorteil, dass das Verfahren bereits bei Erstinbetriebnahmen angewendet werden kann, und auch dann, wenn noch keine Betriebsdaten vorliegen. Dies wird möglich, indem die Datenbasis mit Referenzdaten, also mit Testdaten, die in einer Entwicklungsphase gesammelt werden, zusätzlich mit simulierten Daten erweitert wird.

In einer anderen, vorteilhaften Ausführungsform des Verfahrens ist der Identifikationsalgorithmus als selbstlernendes System ausgebildet. Dazu wird der berechnete Austauschdatensatz für die jeweilige Fehlermeldung (iterativ und kontinuierlich) - und optional: ein zugeordneter Bewertungsdatensatz - in das Datenbanksystem eingepflegt. Damit kann die Qualität des Ergebnisses verbessert werden.

Die Aufgabe wird weiterhin gelöst mit einem Datenbanksystem, welches einen Prozessor zu einer Ausführung des oben beschriebenen Verfahrens und eine Fehlermustererzeugungseinheit umfasst. In einer vorteilhaften Ausführungsform des Datenbanksystems liest die Fehlermustererzeugungseinheit zur Erzeugung der Fehlermuster als Eingangsgrößen aggregierte Testdaten eines Testbetriebs mit Fehlerfällen des medizinischen Gerätes und zugeordneten Austauschdatensätzen ein, um daraus Fehlermuster zu erzeugen. Die Testdaten werden somit in einer Entwicklungsphase des Gerätes und insbesondere noch vor Gerätefreigabe erfasst. Vorzugsweise werden den jeweiligen Fehlerfällen im Testbetrieb (Testdaten), jeweils ein Austauschdatensatz (der das testweise ersetzte Bauteil eindeutig referenziert) und ein Bewertungsdatensatz zugeordnet und als Muster gespeichert. Der Bewertungsdatensatz dient dazu, die Qualitätsbewertung der Fehlerbehebungsmaßnahme (durch den Austausch) zu kennzeichnen.

Parallel zum Testbetrieb des Gerätes wird ein Simulationsmodell erzeugt. Das Simulationsmodell dient zur Erweiterung der Datenbasis mit Simulationsdaten, die aus den bisher aggregierten Daten (Testbetrieb und später auch im realen Gerätebetrieb mit Betriebsdaten) errechnet werden. Die aggregierten Daten umfassen ein Datenfeld zur Kennzeichnung des jeweiligen Fehlerfalls, zugeordneten Austauschdatensätzen und Bewertungsdatensätzen. Diese aggregierten Daten können auch von anderen Geräten vom selben Typ (z.B. dieselbe Modalität oder ein Gerät mit vordefinierbaren übereinstimmenden technischen Kriterien) stammen. Gemäß einer vorteilhaften Ausführungsform der Erfindung ist es vorgesehen, dass die Testdaten auch von anderen Geräten desselben Typs verwendet werden können. Damit wird die Datenbasis erweitert und es ist möglich, früher bzw. schneller an die Test- bzw. Trainingsdaten zu kommen, um diese für die Berechnungen zu nutzen.

In einer weiteren, vorteilhaften Ausführungsform des Datenbanksystems wird das Simulationsmodell nicht nur während dem Testbetrieb in der Entwicklungsphase laufend erweitert, sondern kann auch noch später im Produktlebenszyklus, nämlich während des realen Betriebs mit Betriebsdaten erweitert werden. Damit kann die Qualität der Fehlerbehebungsmaßnahme durch einen Bauteilaustausch kontinuierlich verbessert werden. Dies wird erreicht, indem die Datenbasis zur Erzeugung der Fehlermuster als selbstlernendes System ausgebildet ist und ständig erweitert wird. Dies hat weiter den Vorteil, dass das automatische Bauteilidentifikationsverfahren im Produktzyklus bereits früher und sogar unmittelbar nach Produktfreigabe ohne das Vorhandensein von Betriebsdaten ausgeführt werden kann. In dieser Ausführungsform der Erfindung wird das Gerät in einer Entwicklungsphase getestet. Dazu werden vordefinierbare Testfälle erzeugt, teilweise auch um auf intendierte Weise Fehler zu erzeugen, die dann erfasst werden. Die zur Beseitigung des erfassten Fehlers (testweise) ausgetauschten Bauteile werden als Austauschdatensatz erfasst und in dem Datenbanksystem gespeichert. Die Güte der Fehlerbeseitigung durch den Bauteilaustausch (mit anderen Worten: Ersatz des fehlerhaften Bauteils durch ein neues oder fehlerfreies Bauteil) wird mittels des Bewertungsdatensatzes bewertet und ebenfalls gespeichert. Für zukünftige Fehlerfälle, stehen dann die vorstehend gesammelten Daten (Fehlermeldung, Austauschdatensatz, Bewertungsdatensatz) auf zugeordnete Weise (nämlich als Fehlermuster) in der Datenbank des Datenbanksystems zur Verfügung. Für eine spezifische Fehlermeldung kann auf Basis der hinterlegten Fehlermuster eine Analyse in den gespeicherten Daten ausgeführt werden, um den für den Fehlerfall geeigneten Austauschdatensatz zu errechnen.

In einer weiteren, vorteilhaften Ausführungsform des Datenbanksystems ist dieses als zentrale Instanz für eine Vielzahl von medizinischen Geräten ausgebildet. Damit kann der Umfang der erzeugten Fehlermuster erweitert und die Aussagekraft des Ergebnisses verbessert werden.

In einer anderen, vorteilhaften Ausführungsform des Datenbanksystems interagiert die Fehlermustererzeugungseinheit mit einer Regelbasis, in der Regeln zum Erzeugen der Fehlermuster abgelegt sind. Die Regeln können vordefiniert sein. Die Regeln sind vorzugsweise dynamisch anpassbar. Damit kann das Verfahren vorteilhafterweise sehr flexibel an die Anwendungs- und Betriebssituation des Gerätes angepasst werden.

Eine Regel könnte zum Beispiel eine Reihenfolge der Bearbeitung erst A dann B sein oder A und B in beliebiger Reihenfolge.

In einem anderen Fall kann die Regel bewirken, dass nur die wahrscheinlichsten Fehlermuster verwendet werden sollen oder systemspezifische oder länderspezifische Vorgaben von Regeln möglich sind.

In einer weiteren, vorteilhaften Ausführungsform des Datenbanksystems berücksichtigt die Fehlermustererzeugungseinheit zur Erzeugung von Fehlermustern Bewertungsdaten bzw. Bewertungsdatensätze, die ein Maß für die Güte der jeweiligen bereits ausgeführten Fehlerbehebung repräsentieren und die bei historischen Bauteilersetzungen zu einer Fehlermeldung erfasst und gespeichert worden sind.

Das Datenbanksystem und/oder die Fehlermustererzeugungseinheit umfassen zum Erzeugen der Fehlermuster einen Prozessor oder interagieren mit diesem, der zur Anwendung von einem Verfahren zum maschinellen Lernen und insbesondere zur Ausführung von Markow-Prozessen (bzw. Markow-Ketten) bestimmt ist.

In einer anderen, vorteilhaften Ausführungsform kann der Prozessor auch zur Anwendung von Methoden des Datamining und/oder zur Anwendung von statistischen und stochastischen Methoden zur Datenverarbeitung dienen.

Die eingangs erwähnte Aufgabe wird weiterhin gelöst durch eine Berechnungseinheit, die insbesondere als zentraler Server ausgebildet sein kann und die zum Anbieten eines Bauteilidentifikationsdienstes dient, der nach einem oben beschriebenen Verfahren betrieben wird und dazu mit dem Datenbanksystem interagiert. Die Berechnungseinheit umfasst folgende Bauteile:
- eine Eingangsschnittstelle, die zum Einlesen von jeweils einer Fehlermeldung eines der medizinischen Geräte bestimmt ist;
- einer Identifikationseinheit, die zum Anwenden eines Identifikationsalgorithmus zur Berechnung eines Austauschdatensatzes ausgebildet ist;
- einer Ausgabeschnittstelle, die zur Ausgabe des berechneten Austauschdatensatzes bestimmt ist.

Die Eingangs- und Ausgangsschnittstelle sind als logische Schnittstellen zu verstehen. Sie dienen und regeln den Austausch von Kommandos und Daten zwischen verschiedenen Prozessen und Komponenten des Datenbanksystems bzw. der Berechnungseinheit.

Die Aufgabe wird weiterhin gelöst durch ein medizinisches System mit über ein Netzwerk verbundenen Einheiten, umfassend:
- Eine Mehrzahl von medizinischen Geräten, insbesondere von MR-Geräten, die jeweils eine Vielzahl von Bauteilen umfassen;
- Eine Berechnungseinheit, wie vorstehend beschrieben;
- Ein Datenbanksystem, wie vorstehend beschrieben.

Die Aufgabe wird weiterhin gelöst durch ein Computerprogrammprodukt zur Identifikation von auszutauschenden (fehlerhaften) Bauteilen eines medizinischen Gerätes, wobei das Computerprogrammprodukt auf einem Datenträger gespeichert ist und Computerprogrammcode zur Ausführung des vorstehend beschriebenen Verfahrens auf einem Prozessor eines Computers umfasst.

Im Folgenden finden sich Definitionen für die in dieser Anmeldung verwendeten Begrifflichkeiten.

Das medizinische Gerät ist eine komplexe medizintechnische Vorrichtung, die aus unterschiedlichen elektronischen, mechanischen und/oder physikalischen Bauteilen besteht. Das medizinische Gerät kann einen rein medizinischen Zweck haben (z.B. Bilderfassung) oder einen technischen Zweck (z.B. Datenkompression, Bildnachverarbeitung, Gerätesteuerung) im medizinischen Umfeld. Das Gerät kann z.B. zur Bildakquisition und/oder zur technischen Verarbeitung im Medizinbereich dienen. Wenn im Folgenden von "Gerät" die Rede ist, dann ist das medizinische Gerät gemeint. Es kann z.B. ein MRT-Gerät, ein CT-Gerät oder dergleichen sein. Das Gerät kann in einem Verbund von Geräten desselben oder von unterschiedlichem Typ in einem Geräteverbund betrieben werden.

Die Bauteile können in das Gerät integriert sein oder mit diesem in Datenaustausch stehen. Die Bauteile können unterschiedlichen Funktionen des Gerätes dienen. Ein Bauteil eines MR-Gerätes kann z.B. ein supraleitender Magnet, ein Kühlaggregat, eine Gradientenspule, eine Oberflächenspule, eine Recheneinheit oder eine Kommunikationsverbindung zum Datenaustausch sein. Die Bauteile sind (insbesondere im Fehlerfall) austauschbar in dem Gerät angeordnet und können durch andere (fehlerfreie) Bauteile desselben Typs ersetzt werden.

Das Simulationsmodell wird rechner- bzw. computer-basiert erzeugt und dient zur Modellierung von Fehlerzuständen des Gerätes zur Suche nach passenden Fehlerbehebungsmaßnahmen durch den Austausch eines oder mehrere Bauteile des Gerätes. Das Simulationsmodell kann ein deskriptives Simulationsmodell zur Fehlerbeseitigung sein. Das Simulationsmodell ist vorzugsweise als kontinuierliches Modell ausgebildet. Es arbeitet ereignisgesteuert, in Abhängigkeit vom Eintreten eines neuen Fehlerfalls (im Testbetrieb oder im realen Betrieb des Gerätes). Durch die Anwendung des Simulationsmodells kann das Ergebnis mit dem Vorschlag für einen Bauteiltausch im Fehlerfall hinsichtlich seiner Qualität optimiert werden.

Ein Simulationsmodell kann z.B. eine Markow-Kette darstellen oder mithilfe eines neuronalen Netzes trainiert werden, welches Übergangswahrscheinlichkeiten von Meldung gelernt hat. Damit können viele Logdateien simuliert werden, die auch mit einer gewissen Wahrscheinlichkeit Fehlermuster enthalten. Diese sind dann unabhängig vom Einzelsystem und enthalten eine Vielzahl von Logdateien-Varianten welche ebenfalls gelernt werden können. Die Optimierung des Simulationsmodells kann durch Training und Vergleich mit dem Testsystem oder dem realen System verbessert werden.

Eine Fehlermeldung ist ein elektronischer Datensatz, der als Nachricht über ein Netzwerk mit anderen Instanzen ausgetauscht werden kann. Die Fehlermeldung dient zur Kennzeichnung eines Fehlers oder eines Fehlerzustandes (Kombination von Fehlern), der an einem Gerät erfasst worden ist. Die Fehlermeldung ist vorzugsweise geräte-spezifisch. Die Fehlermeldung ist jedoch bauteil-unspezifisch, so dass aus einer Analyse der Fehlermeldung in der Regel nicht auf ein fehlerhaftes und auszutauschendes Bauteil geschlossen werden kann. Die Fehlermeldung kann einen spezifischen Aufbau haben, der z.B. einem Standard (z.B. DICOM, Digital Imaging and Communication in Medicine) entsprechen kann. Die Fehlermeldung kann unterschiedliche technische Ursachen haben. Die Fehlermeldung ist üblicherweise gerätespezifisch und kann einen Gerätezustand beschreiben. In der Regel kann der Fehlermeldung nicht unmittelbar und ohne Weiteres eine Fehlerursache entnommen werden. Dies bedeutet, dass in der Regel umfangreiches technisches Wissen notwendig ist, um aus einer Fehlermeldung auf ein auszutauschendes Bauteil zu schließen.

Der Identifikationsalgorithmus dient zur Identifikation eines fehlerhaften Bauteils, das auszutauschen ist, um den Fehlerzustand oder Fehler des Gerätes zu beseitigen. Dazu wird die eingelesene Fehlermeldung als Eingangsdatum analysiert und mit weiteren Datensätzen abgeglichen und z.B. mit Referenzdaten (eines Datenbanksystems) verglichen. Der Identifikationsalgorithmus kann noch weitere Daten als Eingangsdaten berücksichtigen. Der Identifikationsalgorithmus liefert als Ergebnis (Ausgangsdaten) einen Austauschdatensatz. Der Identifikationsalgorithmus ist in Software implementiert und kann auf einem Rechner mit handelsüblichem Betriebssystem ausgeführt werden. Der Identifikationsalgorithmus wird vorzugsweise auf einer Identifikationseinheit ausgeführt. Der Identifikationsalgorithmus kann auch verteilt auf unterschiedlichen Rechnereinheiten ausgeführt werden oder er kann zentral von einem Server als Dienst bereitgestellt werden. Der Identifikationsalgorithmus kann auf allen oder ausgewählten Geräten als Client-Applikation und/oder auf einem zentralen Server als Dienst ausgeführt werden. Der Identifikationsalgorithmus führt eine Analyse auf den gespeicherten Fehlermustern in Hinblick auf die eingelesene Fehlermeldung aus.

Die Identifikationseinheit ist eine Recheneinheit, die zur Ausführung des Identifikationsalgorithmus dient.

Der Austauschdatensatz ist ein elektronischer Datensatz, der nach vordefinierbaren Regeln strukturiert ist und von der Identifikationseinheit berechnet und als Ergebnis bereitgestellt wird. Der Austauschdatensatz kennzeichnet auf eindeutige Weise, welches Bauteil am Gerät fehlerhaft ist bzw. ausgetauscht werden muss, damit der Fehler behoben werden kann. Der Austauschdatensatz referenziert somit eindeutig ein Bauteil eines Gerätes in Bezug auf eine Fehlermeldung. Im einfachsten Fall kann der Austauschdatensatz eine eineindeutige Bauteilnummer oder Materialnummer sein.

In einer bevorzugten Ausführungsform der Erfindung kann der Austauschdatensatz weitere Datenfelder mit Metadaten umfassen, insbesondere ein Validierungsdatenfeld zur Beurteilung der Aussagekraft des berechneten Austauschdatensatzes. Das Validierungsdatenfeld kann insbesondere Daten zur Korrektheit und zum Testabdeckung umfassen. Dies erweist sich insbesondere bei früher Anwendung des Simulationsmodells und, falls statistische Methoden zum Testen angewendet worden sind, als vorteilhaft für die Qualität der Fehlerbeseitigung.

Das Datenbanksystem umfasst eine - insbesondere relationale - Datenbank und ein Datenbankmanagementsystem zur technischen Datenverarbeitung. Es kann einen Datenspeicher und einen Prozessor als Berechnungseinheit umfassen. Das Datenbanksystem umfasst eine Fehlermustererzeugungseinheit.

Die Fehlermustererzeugungseinheit dient zur automatischen und computer-implementierten Erzeugung von Fehlermustern, die in dem Datenbanksystem gespeichert werden. Die Fehlermustererzeugungseinheit kann unmittelbar in dem Datenbanksystem angeordnet sein oder sie kann als separate Instanz über Schnittstellen angeschlossen sein.

Ein Fehlermuster ist eine elektronische Datenstruktur, die automatisch berechnet wird. Ein Fehlermuster wird berechnet durch eine Korrelation von unterschiedlichen Datensätzen, die aus einer Fehlerlog-Datei und/oder anderen Dateien, die vom Gerät bereitgestellt werden, ausgelesen werden und sich auf einen bestimmten Fehlertyp oder auf eine Gruppe von Fehlertypen beziehen. Die Korrelation und die Berechnung des Fehlermusters erfolgt nach bestimmten vordefinierbaren Regeln. Dabei wird auf ein Regelwerk zurückgegriffen, das (in einer Regeldatenbasis gespeichert sein kann und) dazu dient, die unterschiedlichen Fehlermeldungen der unterschiedlichen Geräte zu gruppieren und zu klassifizieren in Fehlertypen (z.B. Meldungen zum Gerätezustand, wie "Energieversorgung fehlt/unterbrochen" oder "fehlender Datensatz") oder in Gruppen von Fehlertypen (z.B. Fehlertypen, die sich auf ein bestimmtes Bauteil oder eine Komponente eines Bauteils beziehen). Dem Fehlertyp oder der Gruppe von Fehlertypen eines Fehlermusters wird zumindest ein Austauschdatensatz zugeordnet. Insofern kann ein Fehlermuster auch als Austauschmuster bezeichnet werden.

Das Fehlermuster kann in einer ersten Ausführungsform als metrisches Muster und in einer zweiten Ausführungsform der Erfindung als Bauteilmuster berechnet werden.

In der ersten Ausführungsform zur Erzeugung der Fehlermuster werden die Daten aus den während des Betriebs (z.B. auch während der Messungen) der Geräte erzeugten Logdateien vorverarbeitet, um diese vorverarbeiteten Daten mit Gerätenachrichten (die einen Gerätezustand repräsentieren, wie z.B. einen Abbruch einer Messung bzw. Bildakquisition) zu korrelieren. Aus den korrelierten Datensätzen können dann Korrelationsinformationen extrahiert werden, so dass auf die vollständigen Ereignisaufzeichnungsdateien der Geräte (eventlog) mit diesen Korrelationsinformationen dann Methoden des Datamining angewendet werden können.

In der zweiten Ausführungsform der Erfindung zur Erzeugung der Fehlermuster werden die Gerätenachrichten direkt mit Gerätezustandsdaten zum Abbruch von Messungen korreliert. Diese Korrelationsinformationen werden dann unter Anwendung von Datamining-Methoden und mit den vollständigen Ereignislog-Dateien der Geräte (eventlogs) verwendet, um die Fehlermuster zu erzeugen. Die Gerätenachrichten repräsentieren einen Betriebszustand des Gerätes und können z.B. Felder für folgende Informationen umfassen: Beginn einer Ausfall- oder Stillstandphase des Gerätes (Downtime_Start), Ende der Ausfall- oder Stillstandphase (Downtime_End), die Identifikationsnummer des jeweiligen Gerätes und die auf den Fehler ausgetauschten Bauteile.

Die erzeugten Fehlermuster können in einer tabellenartigen Datenstruktur gespeichert werden und/oder können auf einer grafischen Benutzeroberfläche in Form einer zweidimensionalen Darstellung ausgegeben werden. Die Fehlermuster können auch einen Bewertungsdatensatz umfassen, der kennzeichnet, wie gut der Fehler durch den erfolgten Bauteilaustausch behoben werden konnte. Die Fehlermuster können z.B. auch so erzeugt werden, dass die jeweiligen Datensätze auf Basis der Bewertungsdaten gruppiert werden, und somit die Reihenfolge der Fehlermuster mit einer Güte der Fehlerbehebung korreliert.

Die Fehlermuster von historischen also bereits ausgeführten Bauteilersetzungen auf zurückliegende Fehlermeldungen, werden alle in dem Datenbanksystem gespeichert. Zusätzlich werden die zugeordneten Austauschdaten (also das jeweils ausgetauschte Bauteil) und optional ein Bewertungsdatensatz, der die Güte der Fehlerbehebung repräsentiert, gespeichert. Diese historischen Daten (von bereits ausgeführten Bauteilersetzungen und diesbezüglichen Bewertungen) werden für die Berechnung von dem jeweils aktuellen Austauschdatensatz (auf die aktuelle Fehlermeldung) mittels des Identifikationsalgorithmus analysiert.

Das Fehlermuster wird mittels der Fehlermustererzeugungseinheit durch Korrelation von Eingangsgrößen erzeugt. Ein Fehlermuster ist vorzugsweise spezifisch für einen bestimmten Gerätetyp (z.B. MR-Gerät vom Typ xyz). Es können somit Fehlermuster für unterschiedliche Gerätetypen aggregiert werden. Das Verfahren zur Erzeugung der Fehlermuster ist auf der Fehlermustererzeugungseinheit implementiert und kann auf einer Recheneinheit (Prozessor, ...) des Datenbanksystems ausgeführt werden oder auf einer externen Recheneinheit, die sich außerhalb des Datenbanksystems befindet.

Die Eingangsgrößen sind bevorzugt ebenfalls in der Datenbank gespeichert. Die Eingangsgrößen umfassen Gerätemeldungen zu fehlerhaften Betriebszuständen eines der Geräte, Austauschdatensätze und Bewertungsdatensätze.

Das System und insbesondere der Identifikationsalgorithmus sind als selbstlernendes System ausgebildet. Das bedeutet, dass die Datenbasis kontinuierlich und ständig erweitert wird und die Qualität der Aussage somit mit jeder neuen Fehlermeldung und Anwendung verbessert wird.

Die Berechnungseinheit kann in Software und/oder in Hardware implementiert werden. Die Berechnungseinheit kann auf einem Prozessor eines Geräteverbundes, eines medizinischen Gerätes oder eines zentralen Servers ausgebildet sein, um den Identifikationsdienst als zentralen Dienst für eine Vielzahl von Geräten bereitstellen zu können. Die Berechnungseinheit ist vorzugsweise mit einer Ausgabeschnittstelle (Browser, GUI, Datenschnittstelle...) zur Ausgabe des Austauschdatensatzes ausgebildet. Vorteilhaferweise kann das Verfahren zur Berechnung des Austauschdatensatzes zentral für eine Vielzahl von mitunter auch unterschiedlichen medizinischen Geräten ausgeführt werden (auch parallel oder alternativ lokal auf einem Gerät mit Netzwerkanbindung zu dem zentral vorgehaltenen Datenbanksystem).

Die Identifikationseinheit entspricht einer hardware-basierten Lösung und dient zur Ausführung des Identifikationsalgorithmus. Ebenso entspricht die Fehlermustererzeugungseinheit einer hardware-basierten Lösung und dient der Ausführung eines Verfahrens zur Erzeugung von Fehlermustern, das in Software implementiert ist. Dabei erwähnte Merkmale, Vorteile oder alternative Ausführungsformen sind ebenso auch auf die anderen beanspruchten Gegenstände zu übertragen und umgekehrt. Mit anderen Worten können auch die gegenständlichen Merkmale und Ansprüche (die beispielsweise auf ein System, eine Berechnungseinheit oder auf ein Computerprogrammprodukt gerichtet sind) mit den Merkmalen weitergebildet sein, die in Zusammenhang mit dem jeweiligen Verfahren beschrieben oder beansprucht sind. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

Eine weitere Aufgabenlösung besteht in einem Computerprogrammprodukt zur Identifikation von auszutauschenden Bauteilen eines medizinischen Gerätes auf eine Fehlermeldung, das als speicherlesbares Medium (tragbarer Datenträger mit dem Computerprogramm) oder als Maschine, die zum Download des Computerprogramms bestimmt ist (z.B. als zentraler Server), ausgebildet sein kann.

Eine weitere Aufgabenlösung sieht ein Computerprogramm vor, mit Computerprogrammcode zur Durchführung aller Verfahrensschritte der oben näher beschriebenen Verfahren (zur Erzeugung von Fehlermustern und zur Identifikation von auszutauschenden Bauteilen auf Basis einer konkreten Fehlermeldung), wenn das Computerprogramm auf einem Computer ausgeführt wird. Dabei ist es auch möglich, dass das Computerprogramm auf einem von einem Computer lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Fig. 1: ein Blockschaltbild eines Systems gemäß einer bevorzugten Ausführungsform der Erfindung;
- Fig. 2: ein Ablaufdiagramm eines Verfahrens zur Identifikation von auszutauschen Bauteilen gemäß einer bevorzugten Ausführungsform der Erfindung;
- Fig. 3: eine schematische Übersichtsdarstellung des Systems mit einer Fehlermustererzeugungseinheit an einem Ausführungsbeispiel und
- Fig. 4: ein Sequenzdiagramm zur Darstellung des Datenaustausches zwischen Datenbanksystem, Berechnungseinheit und medizinischem Gerät und
- Fig. 5: ein Sequenzdiagramm zur Darstellung des Datenaustausches zwischen den medizinischen Geräten, dem Datenbanksystem und der Fehlermustererzeugungseinheit und
- Fig. 6: eine schematische Darstellung eines Fehlermusters mit Datenfeldern.

Im Folgenden wird die Erfindung unter Bezugnahme auf die Figuren näher beschrieben.

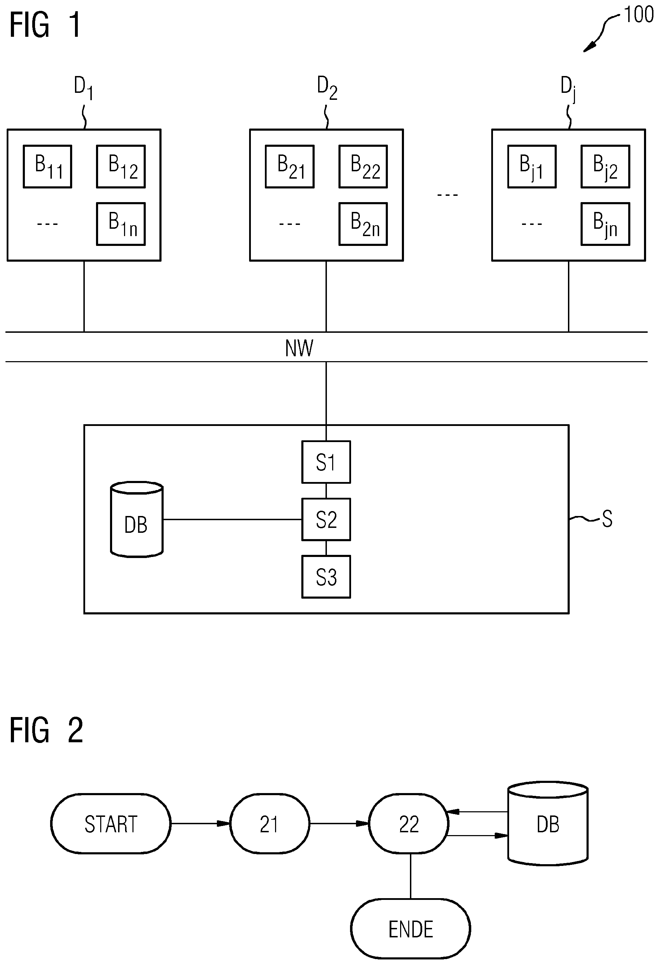

In **Figur 1** ist auf schematische Weise in einer Übersichtsdarstellung ein System 100 dargestellt. Das System 100 umfasst mehrere separate Geräte, die im Folgenden mit den Bezugszeichen D₁, D₂, ... Dⱼ gekennzeichnet sind. Bei den Geräten handelt es sich um technische, insbesondere medizintechnische Geräte, wie beispielsweise Bildakquisitionssysteme, wie unter anderem Magnetresonanztomographen, Computertomographen, Ultraschallgeräte und dergleichen. Wichtig hervorzuheben ist - und für einen Fachmann liegt dies auf der Hand -, dass diese Geräte eine hohe Komplexität aufweisen und eine Vielzahl von Komponenten und Bauteilen B umfassen. Wie in Figur 1 dargestellt, kennzeichnet sich jedes medizinische Gerät D₁, D₂, Dⱼ durch eine Vielzahl von unterschiedlichen Komponenten und Bauteilen, die in Figur 1 mit dem Bezugszeichen B₁₁, B₁₂, B₁ₙ für ein erstes medizinisches Gerät D₁ gekennzeichnet sind und mit Bⱼ₁, Bⱼ₂, ... Bⱼₙ für ein j-tes medizinisches Gerät Dⱼ. Alle medizinischen Geräte sind über ein Netzwerk NW miteinander verbunden und können interagieren bzw. Daten austauschen. Des Weiteren sind die medizinischen Geräte D mit einer Berechnungseinheit S verbunden, der als zentraler Server ausgebildet sein kann und zur Bereitstellung eines Bauteilidentifikationsdienstes dient. Dazu interagiert die Berechnungseinheit S mit einem Datenbanksystem DB, das in der beispielhaften Ausführungsform, die in Figur 1 dargestellt ist, innerhalb der Berechnungseinheit S angeordnet ist. In einer alternativen Ausführungsform kann das Datenbanksystem DB jedoch als externe Instanz über entsprechende Schnittstellen an die Berechnungseinheit S angeschlossen sein (dies wird die bevorzugte Ausführungsform sein). Die Berechnungseinheit S umfasst zum Anbieten des Bauteilidentifikationsdienstes mehrere elektronische Komponenten: Eine Eingangsschnittstelle S1, die zum Einlesen von Fehlermeldungen fm von einem der medizinischen Geräte D bestimmt ist. Darüber hinaus umfasst die Berechnungseinheit S eine Identifikationseinheit S2, die zum Anwenden eines Identifikationsalgorithmus' 22 zur Berechnung eines Austauschdatensatzes ad ausgebildet ist. Weiterhin umfasst die Berechnungseinheit S eine Ausgabeschnittstelle S3, die zur Ausgabe des als Ergebnis berechneten Austauschdatensatzes ad bestimmt wird. Dabei kann es sich beispielsweise um eine grafische Benutzerschnittstelle (graphical user interface - GUI) oder um Schnittstellen zu angeschlossenen Clients und/oder Servern handeln.

Die Berechnungseinheit S dient zum Ausführen eines Identifikationsverfahrens, das im Folgenden unter Bezugnahme auf **Figur 2** näher beschrieben wird.

Nach dem Start des Verfahrens zur Identifikation von auszutauschenden Bauteilen B eines medizinischen Gerätes wird in Schritt 21 jeweils eine Fehlermeldung fm eines der Geräte D eingelesen. Dabei ist das System dahingehend nicht eingeschränkt, wie viele Fehlermeldungen fm pro Zeiteinheit eingehen. Das Verfahren bzw. das vorgeschlagene System ist in der Lage, auch parallel empfangene Fehlermeldungen fm gleichzeitig zu verarbeiten. Das Einlesen in Schritt 21 erfolgt vorzugsweise in einer Betriebsphase des Gerätes, also in der Regel im klinischen Einsatz z.B. eines MR-Gerätes D.

In Schritt 22 erfolgt die Ausführung des Identifikationsalgorithmus zur Berechnung und zur Ausgabe eines Austauschdatensatzes ad, der ein spezifisch auf die erfasste Fehlermeldung fm hin auszutauschendes Bauteil B identifiziert. Der Austauschdatensatz ad ist somit bauteilspezifisch und fehlermeldungsspezifisch. Der Identifikationsalgorithmus 22 kann auf einem beliebigen Computer oder Recheneinheit oder einem Verbund von Recheneinheiten oder einem Prozessor eines Rechners ausgeführt werden. Der Identifikationsalgorithmus 22 greift zur Berechnung auf das Datenbanksystem DB zu, in der Fehlermuster fmu mit Austauschdatensätzen ad und zugeordneten Bewertungsdatensätzen bd abgelegt sind. Der Zugriff auf das Datenbanksystem DB erfolgt dediziert für die eingelesene Fehlermeldung fm, um spezifisch für diese eingelesene Fehlermeldung fm aus den in dem Datenbanksystem DB gespeicherten Fehlermustern fmu einen Austauschdatensatz ad zu berechnen, der der eingelesenen Fehlermeldung fm zugeordnet ist und eine möglichst hohe Güte aufweist, also ein optimales Austauschergebnis in Hinblick auf die Problemlösung hat. Damit kann sichergestellt werden, dass das Ergebnis des Identifikationsalgorithmus 22 möglichst nur diejenigen Bauteile B indiziert, die für den in der Fehlermeldung fm gekennzeichneten Fehler ursächlich sind und bei einem Austausch bzw. Ersatz eine Fehlerbehebung zur Folge haben. Das Ergebnis des Identifikationsalgorithmus 22 kann in einem nachfolgenden Verfahrensschritt und insbesondere auf einer Ausgabeschnittstelle ausgegeben oder zum Abruf bzw. Download bereitgestellt werden.

Wie vorstehend bereits erläutert, sind in dem Datenbanksystem DB Fehlermuster fmu gespeichert, die zur Berechnung von Austauschdatensätzen ad dienen. Ein wichtiger Aspekt der vorliegenden Erfindung besteht deshalb darin, die Erzeugung der Fehlermuster fmu in dem Datenbanksystem DB auszuführen. Dies wird im Folgenden näher erläutert.

Zur Erzeugung der Fehlermuster fmu ist eine Fehlermustererzeugungseinheit FME vorgesehen. Diese kann als computerbasierte Einheit umfassend Hardware und Software ausgebildet sein. Die Fehlermustererzeugungseinheit FME kann somit auch als Prozessor ausgebildet sein, der zur Ausführung eines Algorithmus zur Fehlermustererzeugung dient. Der Algorithmus zur Fehlermustererzeugung basiert auf Methoden des maschinellen Lernens.

Die Fehlermustererzeugung greift auf das Simulationsmodell zu. Das Simulationsmodell kennzeichnet sich in der Regel durch das Erzeugen von Simulationsdaten auf Basis von erfassten Daten (also Daten aus einem Testbetrieb oder einem Gerätebetrieb im Feld). Das Simulationsmodell dient dazu, erlernte Daten, Muster und Gesetzmäßigkeiten anzuwenden, um neue Daten (simulierte Daten) zu erzeugen. Die erfindungsgemäß eingesetzten Methoden des maschinellen Lernens basieren auf Eingangsgrößen, die aus historisch erfassten Fehlermeldungen unterschiedlicher medizinischer Geräte D extrahiert worden sind. Die Eingangsgrößen umfassen zum einen die jeweilige Fehlermeldung des jeweiligen medizinischen Gerätes D, das im Hinblick auf diese Fehlermeldung ausgetauschte Bauteil. Als wichtiges, weiteres Merkmal umfassen die Eingangsgrößen zusätzlich noch Bewertungsdaten bd (bzw. einen Bewertungsdatensatz), die als Maß für die Güte der bereits ausgeführten (früheren) historischen Fehlerbehebung durch Austausch des jeweiligen Bauteiles B dienen. Mit anderen Worten kennzeichnen die Bewertungsdaten bd, wie gut sich der jeweilige Fehler (in der Fehlermeldung fm repräsentiert) durch den Austausch des jeweiligen Bauteils B beheben ließ. Die Qualität bzw. die Effizienz der historischen Fehlerbehebungsmaßnahme fließt somit in die Erzeugung der Fehlermuster der Fehlermustererzeugungseinheit FME ein. Damit wird ein kontinuierlicher und iterativer Lernprozess sichergestellt.

Die Fehlermustererzeugungseinheit FME ist ausgebildet, die Fehlermuster fmu durch Korrelation der (kontinuierlich neu eingepflegten) Eingangsgrößen zu erzeugen. Ein Fehlermuster fmu, das entsprechend erzeugt worden ist, umfasst eine Zuordnung zwischen einem Fehlertyp (zum Beispiel vollständiger oder teilweiser Ausfall eines Bauteils B oder teilweise gestörte Kommunikationsverbindung etc.) oder einer Kombination aus Fehlertypen und einem Austauschdatensatz ad. Der Austauschdatensatz ad bezieht sich somit auf historische Austausche, die aufgrund einer Fehlermeldung fm ausgeführt worden sind. Der Austauschdatensatz ad kennzeichnet das damals ausgetauschte Bauteil. Wie oben bereits erwähnt, repräsentieren die erzeugten Fehlermuster fmu zusätzlich noch eine Einstufung der Güte der damaligen Fehlerbehebung, die in den Bewertungsdaten bd repräsentiert ist. Aus diesen Eingangsgrößen erstellt die Fehlermustererzeugungseinheit FME Fehlermuster. Diese werden in dem Datenbanksystem DB kontinuierlich gespeichert und mit jeweils neuen Lernvorgängen angereichert. Die so erzeugten und gespeicherten Fehlermuster fmu sind somit in dem Datenbanksystem DB abgelegt und werden von dem Identifikationsverfahren verwendet, um den Austauschdatensatz ad zu berechnen.

In einer bevorzugten Ausführungsform der Fehlermustererzeugungseinheit FME ist diese ausgebildet, computer-basierte, automatische Mustererkennungsverfahren anzuwenden. Auf den eingelesenen Daten bzw. auf den Eingangsgrößen (wie vorstehend erläutert) können Markow-Prozesse angewendet und zur Erzeugung der Fehlermuster fmu verwendet werden.

Vorzugsweise werden die Mustererkennungsverfahren bzw. die Verfahren des maschinellen Lernens auf einem Prozessor P ausgeführt. Der Prozessor P kann darüber hinaus noch weitere Algorithmen ausführen, wie beispielsweise Methoden des Datamining und/oder statistische Methoden der Datenverarbeitung.

In einer bevorzugten Ausführungsform der Erfindung ist es vorgesehen, dass bestimmte Regeln zur Erzeugung der Fehlermuster fmu bereitgestellt werden. Damit soll sichergestellt werden, dass die zu erzeugenden Fehlermuster fmu möglichst optimal an die aktuellen technischen Gegebenheiten der medizinischen Geräte angepasst sind und, dass damit die Qualität des Ergebnisses mit dem Austauschdatensatz ad möglichst hoch ist. Dazu ist es vorgesehen, dass die Fehlermustererzeugungseinheit FME mit einer Regelbasis RDB interagiert, in der Regeln zum Erzeugen der Fehlermuster fmu abgelegt sind. Die Regeln können auch noch während des Betriebs, also auch bereits während des Einsatzes der Geräte im Feld (Gerätebetriebsphase), dynamisch geändert oder angepasst werden. Damit kann die Erzeugung der Fehlermuster fmu sehr aktuell und dynamisch auf die jeweilige technische Situation abgestellt werden (z.B., wenn das Gerät in einer neuen Umgebung eingesetzt wird oder mit einem neuen Betriebssystempatch betrieben werden soll).

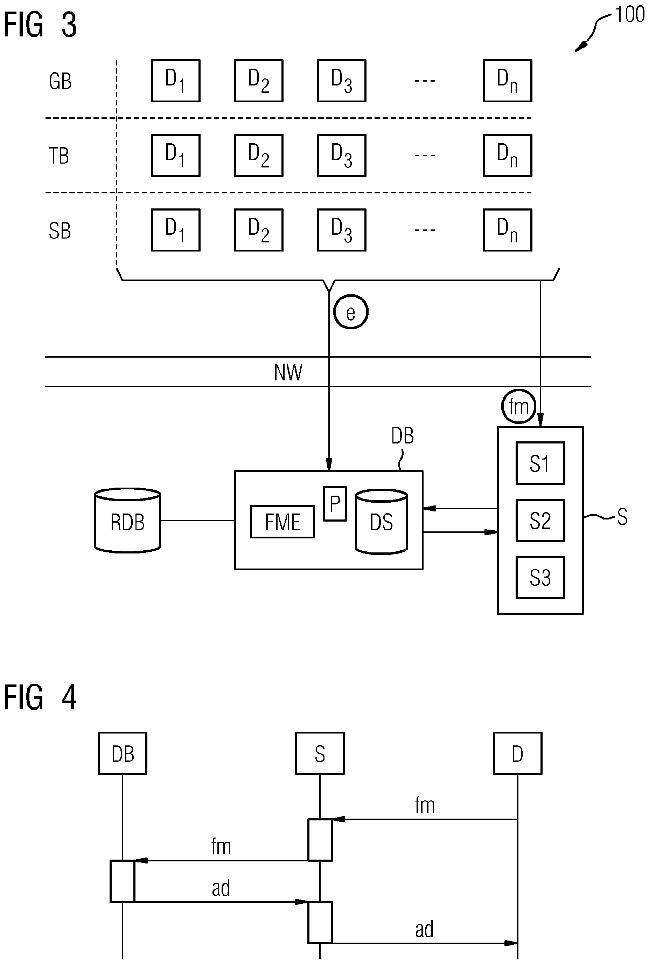

In **Figur 3** ist im unteren Bereich die Berechnungseinheit S mit ihren weiteren Komponenten und das Datenbanksystem DB mit der Regelbasis RDB dargestellt. In dem hier dargestellten Beispiel ist die Regelbasis RDB als separate Datenbank dargestellt, die über eine entsprechende Schnittstelle mit dem Datenbanksystem DB verbunden ist. Diese Ausführungsform ist jedoch nicht zwingend. Ebenso kann es sein, dass die Regelbasis RDB in das Datenbanksystem DB integriert ist. In dieser beispielhaften Ausführungsform ist es ferner vorgesehen, dass das Datenbanksystem DB einen Datenspeicher DS, den Prozessor P und die Fehlermustererzeugungseinheit FME als separate elektronische Instanzen umfasst. Auch dies ist nicht zwingend erforderlich. In alternativen Ausführungsformen können die vorstehend genannten Bauteile FME, P, DS auch in ein einheitliches Modul des Datenbanksystems DB integriert werden. Alternativ können die vorstehenden Komponenten auch auf separate, eigenständige Komponenten verteilt werden, so dass beispielsweise die Fehlermustererzeugungseinheit FME auf einer separaten Recheneinheit ausgebildet ist, die über eine Datenverbindung mit dem Datenbanksystem DB in Datenaustausch steht. Selbiges gilt auch für den Prozessor P.

Wie in Figur 3 schematisch dargestellt, umfasst das erfindungsgemäße System 100 die medizinischen Geräte D, die über das Netzwerk NW mit dem Datenbanksystem DB und mit der Berechnungseinheit S in Datenaustausch stehen.

Wie vorstehend bereits erläutert, basiert das Verfahren zur Identifikation von auszutauschenden bzw. zu ersetzenden Bauteilen B auf automatisch generierten Fehlermustern fmu, die in dem Datenbanksystem DB abgelegt sind. Grundsätzlich sind somit zwei Zeitphasen zu unterscheiden:
1. Eine Anwendungsphase. In dieser Phase wird das Verfahren zur Identifikation von auszutauschenden Bauteilen B ausgeführt. Diese Phase entspricht üblicherweise einer Betriebsphase des jeweiligen medizinischen Gerätes D. Sobald das medizinische Gerät D z.B. im klinischen Einsatz auf einen Fehler läuft, wird eine Fehlermeldung fm erzeugt, die über die Schnittstelle an die Berechnungseinheit S weitergeleitet wird, damit diese das auszutauschende Bauteil B berechnen kann, um den Fehler möglichst schnell und effizient zu beheben. Dazu greift die Berechnungseinheit S auf die Fehlermuster und das Simulationsmodell zurück, die in dem Datenbanksystem vorgehalten sind.
2. Fehlermustererzeugungsphase. Diese Phase dient zur Erzeugung von Fehlermustern, die in dem Datenbanksystem DB abgespeichert werden sollen. Diese Phase ist sozusagen die Voraussetzung, damit das Bauteilidentifikationsverfahren (in der Anwendungsphase nach Punkt 1) ausgeführt werden kann, weil die Berechnungseinheit S auf die Fehlermuster fmu zugreifen muss. Diese Phase ist deshalb der Anwendungsphase zeitlich vorangestellt. Diese Phase kann mit einer Simulationsphase zur Erstellung des Simulationsmodells übereinstimmen. Die Fehlermustererzeugungsphase kann aber auch mit einer Testphase für das medizinische Gerät D übereinstimmen.

Da das Verfahren zur Identifikation von auszutauschenden Bauteilen B als selbstlernendes System ausgebildet ist, können auch noch während der Anwendungsphase weitere Daten als Eingangsgrößen für die Fehlermustererzeugungseinheit FME gesammelt und aggregiert werden.

Die unterschiedlichen Zeitphasen sollen in Figur 3 schematisch zusammengefasst werden. In einer bevorzugten Ausführungsform wird zwischen drei Phasen unterschieden:
1. Einer Gerätebetriebsphase GB,
2. einer Testbetriebsphase TB, und
3. einer Simulationsphase bzw. einem Simulationsbetrieb SB.

Die Gerätebetriebsphase bezieht sich auf einen normalen Gerätebetrieb im Feld nach Auslieferung bzw. Produktfreigabe des medizinischen Gerätes D. Üblicherweise wird in dieser Gerätebetriebsphase das Verfahren zur Identifikation von auszutauschenden Bauteilen B angewendet. Dies entspricht der Anwendungsphase (siehe oben). Hier können Fehlermeldungen fm an den Geräten D erfasst und an die Berechnungseinheit S weitergereicht werden, wie dies in Figur 3 auf der rechten Seite durch den nach unten weisenden mit dem Bezugszeichen fm für eine Fehlermeldung gekennzeichneten Pfeil repräsentiert sein soll.

Die Testbetriebsphase TB dient einem Testbetrieb des medizinischen Gerätes D, insbesondere vor Produktfreigabe bzw. Auslieferung des Gerätes D. Die Testbetriebsphase TB findet somit noch in einem Entwicklungszeitraum statt. Im Testbetrieb TB werden dezidiert Fallkonstellationen auf dem jeweiligen Gerät D ausgeführt, um sozusagen "bewusst" Fehler zu evozieren. Diese Fehler führen infolge zur Ausgabe von Fehlermeldungen fm. In dieser Phase können quasi versuchsweise Fehlermuster fmu erzeugt und entsprechend Austauschdaten ad generiert werden, die dann einer Bewertung unterworfen werden. Die Bewertungsdaten werden in Form von Bewertungsdatensätzen bd in einem Speicher des Datenbanksystems DB vorgehalten. In dieser Phase können die erzeugten Fehlermuster fmu einer Überprüfung bzw. Validierung unterzogen werden. Diese Validierung bzw. Bewertung erfolgt automatisch oder manuell. Bei der automatischen Validierung können vordefinierbare Regeln zum Einsatz kommen, die vordefinierte Parameter auswerten, beispielsweise die Dauer einer Ausfallzeit messen bzw. die Zeitspanne zwischen Fehlermeldung und Fehlerbeseitigung bei dem jeweils ersetzten Bauteil. Eine Regel kann die Fehlerbehebungsmaßnahme (über den Bewertungsdatensatz) somit nach der Dauer der Ausfallzeit optimieren. Der Bewertungsdatensatz bd repräsentiert somit dann ein besseres Qualitätsmaß, wenn der Fehler durch den jeweiligen Bauteiltausch schneller behoben werden konnte. Eine andere Regel kann den Umfang der Fehlerbehebung repräsentieren. Der Bewertungsdatensatz bd repräsentiert in diesem Fall dann ein besseres Qualitätsmaß, wenn der Fehler durch den jeweiligen Bauteiltausch umfassender (und z.B. nicht nur teilweise) behoben werden konnte. Vorzugsweise sind in dem Datenbanksystem DB deshalb nur solche Fehlermuster fmu abgelegt, die ein voreingestelltes ausreichendes Maß an Qualität aufweisen. Das Qualitätsmaß kann dabei in einer vorgeschalteten Definitionsweise vom Anwender gewählt werden.

Der Simulationsbetrieb SB der medizinischen Geräte D ist kein realer Betrieb der Geräte, sondern dient lediglich zum Aufbau und zur Anwendung des Simulationsmodells zur Erzeugung einer erweiterten Datenbasis, die wiederum zur Erzeugung der Fehlermuster fmu verwendet wird. Der Simulationsbetrieb kann in zeitlicher Hinsicht mit der Testbetriebsphase übereinstimmen. Der Simulationsbetrieb bringt den technischen Vorteil, dass die Bauteilidentifikation auch bereits dann ausgeführt werden kann, wenn noch keine Betriebsdaten mit realen Daten zu Bauteilersetzungen vorliegen. Der Simulationsbetrieb dient zur Vorhersage und zum Lernen von neuen Mustern auf Basis von Testdaten, die in der Entwicklungsphase gesammelt werden.

Wie in Figur 3 schematisch dargestellt, werden in allen drei Phasen Gerätebetriebsphase GB, Testbetriebsphase TB und Simulationsbetrieb SB Eingangsgrößen der medizinischen Geräte D erfasst und über das Netzwerk NW an das Datenbanksystem DB und insbesondere an die Fehlermustererzeugungseinheit FME zur Datenverarbeitung weitergeleitet. Diese Implementierung, die auf einem selbstlernenden System basiert, hat den technischen Vorteil, dass auch bereits dann eine ausreichende Datenbasis mit Fehlermustern fmu bereitgestellt werden kann, wenn das jeweilige medizinische Gerät D erstmals in Betrieb genommen wird und damit noch keine Gerätebetriebsdaten vorliegen. Damit kann das Verfahren zur Identifikation von auszutauschenden Bauteilen auch ohne Betriebsdaten unmittelbar nach einer erstmaligen Betriebsaufnahme des medizinischen Gerätes ausgeführt werden, da auf Fehlermuster fmu zurückgegriffen werden kann, die in einer Testbetriebsphase TB und durch ein Simulationsmodell zurückgegriffen werden kann.

In Figur 3 werden die Eingangsgrößen mit dem Bezugszeichen e gekennzeichnet. Diese werden in den unterschiedlichen Phasen GB, TB, SB von den jeweiligen medizinischen Geräten D über das Netzwerk NW erfasst und insbesondere an die Fehlermustererzeugungseinheit FME weitergegeben. Die Eingangsgrößen e dienen zur Erzeugung der Fehlermuster fmu. In Figur 3 ist ebenfalls gekennzeichnet, dass die medizinischen Geräte in der Anwendungsphase, also während des realen Gerätebetriebs im Feld, im Fehlerfall Fehlermeldungen fm erzeugen, die ebenfalls über dasselbe oder über ein anderes Netzwerk an die Berechnungseinheit S weitergeleitet werden, damit diese einen Austauschdatensatz ad unter Zugriff auf das Datenbanksystem DB erzeugt, damit die lokale Einheit (Modalität) weiß, welches Bauteil B auszutauschen (zu ersetzen) ist, damit der gemeldete Fehler beseitigt werden kann.

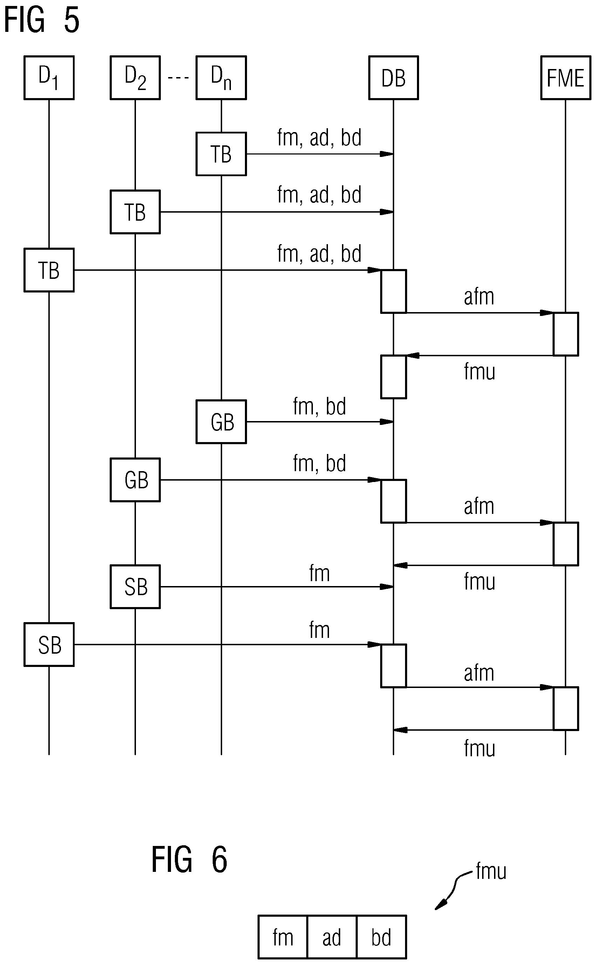

In **Figur 4** ist ein Sequenzdiagramm dargestellt, das die beteiligten Interaktionen zwischen dem Datenbanksystem DB, der Berechnungseinheit S und dem jeweiligen medizintechnischen Gerät D repräsentieren soll. Die in Figur 4 dargestellten ausgetauschten Nachrichten beziehen sich auf eine Anwendungsphase, also auf eine Phase, in der sich das jeweilige medizinische Gerät D im realen Betrieb befindet. Im Fehlerfall erzeugt das Gerät D eine Fehlermeldung fm, die vom Gerät D an die Berechnungseinheit S gesendet wird. Die Berechnungseinheit S liest diese Fehlermeldung fm ein und greift mit dieser Fehlermeldung fm auf das Datenbanksystem DB zu, um die dort abgelegten Fehlermuster fmu zu analysieren, um den zu der jeweiligen Fehlermeldung fm zugeordneten Austauschdatensatz ad zu berechnen. Der mittels einer Musteridentifikationsanalyse berechnete Austauschdatensatz ad wird dann von dem Datenbanksystem DB an die Berechnungseinheit S weitergeleitet und kann dort unmittelbar auf einer Benutzerschnittstelle ausgegeben werden und/oder er kann an das jeweilige lokale, medizinische Gerät D zurückgesendet werden, um dort die geeigneten Fehlerbehebungsmaßnahmen durch die Identifikation des auszutauschenden Bauteils anzustoßen. Die Musteridentifikationsanalyse ist in dem Identifikationsalgorithmus implementiert und basiert auf dem Simulationsmodell. Das Vorstehende, in Zusammenhang mit Figur 4 beschriebene Verfahren setzt voraus, dass bereits Fehlermuster fmu durch die Fehlermustererzeugungseinheit FME erzeugt worden sind. Die Fehlermustererzeugung wird nachstehend in Zusammenhang mit **Figur 5** näher erläutert.

Figur 5 zeigt den Nachrichtenaustausch zwischen den einzelnen medizinischen Geräten D, dem Datenbanksystem DB und der Fehlermustererzeugungseinheit FME, um die Fehlermuster zu erzeugen.

Auf den medizinischen Geräten D₁, D₂ ... Dₙ werden in unterschiedlichen Zeitphasen Eingangsgrößen erfasst. Zum Zwecke der Fehlermustererzeugung und dem Erzeugen einer Datenbasis können die Geräte D sowohl in einem Testbetrieb TB mit Testdaten und mit Simulationsdaten betrieben werden. Darüber hinaus können im realen Feld (Gerätebetrieb GB) auch reale Daten (Fehlermuster mit Austauschdaten und Bewertungen) in das Datenbanksystem eingepflegt werden, um die Datenbasis kontinuierlich zu erweitern. Dabei können die unterschiedlichen Geräte D in jeweils ein und demselben Zeitraum in unterschiedlichen Phasen betrieben werden (Test/Simulation und Gerätebetrieb) und somit einen unterschiedlichen Typ von Eingangsgrößen erfassen.

Zur Initialisierung des Simulationsmodells können im Fehlerfall einer Fehlermeldung initiale Austauschdatensätze ad zugeordnet werden, die dann einer Bewertung unterzogen werden. Je nach Qualität der Fehlerbehebungsmaßnahme werden dann Bewertungsdatensätze bd erzeugt, in denen die Güte der jeweiligen Austauschdaten ad repräsentiert ist. Auf einer abstrakten Ebene kann festgehalten werden, dass bei einem ausreichenden Gütemaß die Zuordnung zwischen Fehlermeldung, und Austauschdaten als Fehlermuster fmu in dem Datenbanksystem DB gespeichert wird. Bei nicht ausreichender Güte wird die jeweilige Zuordnung zwischen Fehlermeldung und Austauschdatensätzen nicht als Fehlermuster fmu gespeichert. Damit kann sichergestellt werden, dass nur eine Auswahl von Fehlermustern in dem Datenbanksystem DB gespeichert wird und nur solche Fehlermuster fmu umfasst, die ein vordefinierbares Gütemaß haben.

In einer Gerätebetriebsphase GB werden wiederum Eingangsgrößen auf den medizinischen Geräten D erfasst, die an das Datenbanksystem DB weitergeleitet werden. In dieser Phase kann die Datenbasis mit realen Daten erweitert werden, indem im Gerätebetrieb GB reale Fehlermeldungen fm erfasst werden und dann von dem Datenbanksystem DB an die Fehlermustererzeugungseinheit FME in aggregierter Form als aggregierte Fehlermeldungen afm weitergeleitet werden. Daraufhin kann die Fehlermustererzeugungseinheit FME Fehlermuster fmu aus den aggregierten Fehlermeldungen afm erzeugen und diese an das Datenbanksystem DB übertragen.

Durch Anwendung von maschinellen Lernverfahren und einem Simulationsbetrieb SB ist es möglich, auch bereits bei Produktfreigabe, eine ausreichende Datenbasis für die Fehlermuster fmu bereitstellen zu können. Dies ist selbst dann möglich, wenn das jeweilige Gerät D noch nicht im realen Feld (Gerätebetrieb GB) betrieben werden kann. Dann greift das Verfahren auf die vorliegenden Fehlermuster fmu zu, die im Testbetrieb TB und/oder im Simulationsbetrieb SB erzeugt worden sind.

**Figur 6** zeigt eine schematische Darstellung eines Fehlermusters fmu. Ein Fehlermuster kann in einer tabellarischen Datenstruktur gespeichert werden, wie in Figur 6 schematisch dargestellt. Ein Fehlermuster fmu umfasst eine Zuordnung zwischen jeweils einer Fehlermeldung fm, einem Austauschdatensatz ad und einem Bewertungsdatensatz bd. Dabei kann eine Fehlermeldung fm nur genau einem Austauschdatensatz ad zugeordnet sein oder eine Fehlermeldung fm kann auch mehreren Austauschdatensätzen ad zugeordnet sein. Der letzte Fall kennzeichnet die Situation, dass bei einer Fehlermeldung fm mehrere Bauteile B auszutauschen sind, damit der Fehler behoben werden kann. Ebenso können auch mehrere Fehlermeldungen denselben Austauschdatensatz ad aufweisen. Dies bedeutet, dass bei unterschiedlichen Fehlermeldungen der Austausch ein und desselben Bauteils B zur Fehlerbehebung führt. Wie vorstehend bereits beschrieben repräsentieren die Bewertungsdaten bd die Qualität des Austauschs bzw. der erzeugten Austauschdaten ad.

Ein wichtiger Vorteil des erfindungsgemäßen Vorschlags ist darin zu sehen, dass die gelernten Fehlermuster, die auf einer ersten Menge von medizinischen Geräten gelernt worden ist, automatisch auch auf eine zweite Menge von medizinischen Geräten übertragen werden kann. Technisch wird dies möglich, indem zu der jeweiligen Fehlermeldung fm und dem Austauschdatensatz ad zusätzlich noch der Bewertungsdatensatz bd mitgeführt wird. Durch die Verarbeitung des Bewertungsdatensatzes bd wird eine kontinuierliche Verbesserung der erzeugten Fehlermuster möglich. Das maschinelle Lernverfahren erzeugt über die Jahre während des Gerätebetriebs GB immer häufigere und bessere Fehlermuster fmu. Der erfindungsgemäße Vorschlag dient dazu, die Qualität von Fehlerbehebungsmaßnahmen insgesamt zu steigern, da die auszutauschenden Bauteile B eindeutig identifizierbar sind und dient zum anderen zur Verringerung von Maintenance-Kosten, da nun automatisch ein Vorschlag zum Bauteiltausch erzeugt werden kann, ohne dass ein Servicetechniker vor Ort notwendig ist.

Durch das Mitführen und Verarbeiten von Bewertungsdaten bd zu den erzeugten Fehlermustern fmu können die Fehlermuster über den Verlauf der Zeit kontinuierlich gelernt und verbessert werden.

In einer bevorzugten Ausführungsform wendet der Identifikationsalgorithmus 22 mathematische und stochastische Optimierungsverfahren an, um die Qualität des Ergebnisses zu verbessern. In einer vorteilhaften Ausführungsform ist es deshalb vorgesehen, dass der Austauschdatensatz ad ein zusätzliches Feld der Metadaten umfasst. Die Metadaten können Angaben über die Optimierungsmethoden umfassen, so kann insbesondere das Maß einer Testabdeckung (coverage) und das Maß einer Korrektheit (correctness) angegeben werden. Dies hat den Vorteil, dass ein Client am medizinischen Gerät D aus den erzeugten Austauschdaten ad unmittelbar erkennen kann, wie die Qualität des jeweiligen Austauschdatensatzes ad bewertet wurde und ob insbesondere eine hohe oder geringe Testabdeckung und/oder Korrektheit vorliegen.

Soll das Verfahren beispielsweise für MR-Geräte eingesetzt werden, so umfassen diese Geräte über 500 Bauteile B, so dass die Datenmenge pro simuliertem Systemtag eines MR-Gerätes D bei 30.000 Fehlermeldungen liegt. Geht man dann davon aus, dass parallel 100 MR-Geräte für einen Simulationsbetrieb SB berücksichtigt werden, so kann man bei 100 Tagen von 300 Millionen Fehlermeldungen ausgehen und damit bis zu 1 Million Bauteilersetzungen pro Systemfamilie simulieren. Wie vorstehend bereits erläutert, können die Daten und die gelernten Daten von unterschiedlichen Geräten D innerhalb einer Systemfamilie (gleicher Gerätetyp) auch für andere Geräte derselben Systemfamilie verwendet werden. Damit kann vorteilhafterweise die Basis für das maschinelle Lernverfahren erweitert werden.

Ein Fehlermuster fmu wird automatisch aufgrund von aggregierten Eingangsgrößen erzeugt. Ein Fehlermuster fmu umfasst zu einer spezifischen Fehlermeldung fm den berechneten Austauschdatensatz ad und einen Bewertungsdatensatz bd, gegebenenfalls mit weiteren Metadaten zur Testabdeckung und zur Korrektheit. Deshalb kann ein erzeugtes Fehlermuster fmu auch als Bauteiltauschmuster bezeichnet werden. Ein Fehlermuster fmu repräsentiert eine Gruppe von Fehlermeldungen fm, Austauschdatensätzen ad, die nach vordefinierbaren Regeln (z.B. Maß der Qualität der Fehlerbehebung) gruppiert worden sind.

In einer Initialisierungsphase zum Aufbau des Simulationsmodells wird selbiges mit bereits erfolgten, historischen Bauteilersetzungen und diesbezüglichen Datensätzen und mit Testdaten als Eingangsgrößen gespeist. Bei allen Fehlermeldungen und nachfolgenden Bauteilersetzungen werden somit Daten erhoben, die insbesondere folgende Datenelemente umfassen:
- Fehlermeldung bzw. Typ der Fehlermeldung fm
- zugeordneter Austauschdatensatz ad
- Bewertungsdatensatz bd
- optional: Metadaten, umfassend Daten zur Korrektheit und Testabdeckung.

Bei einer Fehlermeldung und nachfolgendem Bauteiltausch wird erfasst, wie gut sich der Fehler durch den Bauteiltausch beheben ließ. Diese Daten werden kontinuierlich und fortlaufend in das Datenbanksystem DB eingepflegt. Aus diesen Daten können durch Verfahren des maschinellen Lernens, Prognosen für zukünftige noch nicht bekannte Daten berechnet werden. Auf Basis des Simulationsmodells kann das System auch für neue Fehlermeldungen fm Austauschdatensätze ad prognostizieren.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Für einen Fachmann ist es insbesondere offensichtlich, dass die Erfindung nicht nur für MR-Geräte angewendet werden kann, sondern auch für andere medizinische Geräte, bei denen im Fehlerfall Bauteile ausgetauscht werden müssen. Des Weiteren können die Bauteile des Systems auf mehrere physikalische Produkte verteilt realisiert werden.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

## Patentansprüche

1. Verfahren zur Identifikation von auszutauschenden Bauteilen (B) eines medizinischen Gerätes (D), das jeweils eine Vielzahl von Bauteilen umfasst, insbesondere eines MR-Gerätes, mit folgenden Verfahrensschritten:
- Einlesen (21) von einer Fehlermeldung (fm) des Geräts (D);
- Ausführen eines Identifikationsalgorithmus (22) zur Berechnung und Ausgabe eines ein auszutauschendes Bauteil (B) identifizierenden Austauschdatensatzes (ad) für die eingelesene Fehlermeldung (fm), wobei der Identifikationsalgorithmus (22) mit der eingelesenen Fehlermeldung (fm) auf ein Datenbanksystem (DB) zugreift, in der Fehlermuster (fmu) mit Austauschdatensätzen abgelegt sind, die aus einem Simulationsmodell berechnet wurden, um als Ergebnis denjenigen Austauschdatensatz (ad) zu berechnen, der der eingelesenen Fehlermeldung (fm) zugeordnet ist, wobei in dem Datenbanksystem (DB) Fehlermuster (fmu) gespeichert sind, die automatisch mittels einer Fehlermustererzeugungseinheit (FME) erzeugt werden, wobei die Fehlermustererzeugungseinheit (FME) zur Ausführung eines maschinellen Lernverfahrens auf Basis eines Simulationsmodells ausgebildet ist, das zum kontinuierlichen Lernen und Speichern von Fehlermustern (fmu) durch Korrelation von Eingangsgrößen (e) dient, wobei jeweils ein Fehlermuster (fmu) eine Zuordnung umfasst zwischen einem Fehlertyp oder einer Kombination aus Fehlertypen und einem Austauschdatensatz (ad), der ein auszutauschendes Bauteil (B) indiziert und bei dem das Datenbanksystem (DB) oder die Fehlermustererzeugungseinheit (FME) zum Erzeugen der Fehlermuster (fmu) einen Prozessor (P) umfasst oder mit diesem in Datenaustausch steht, wobei der Prozessor (P) zur Erzeugung der Fehlermuster (fmu) zur Anwendung von Markow-Prozessen ausgebildet ist.

2. Verfahren nach Anspruch 1, bei dem das Ausführen des Identifikationsalgorithmus (22) durch das Einlesen (21) der Fehlermeldung (fm) getriggert wird.

3. Verfahren nach einem der vorstehenden Ansprüche, bei dem das Verfahren ohne Betriebsdaten und unmittelbar nach Betriebsaufnahme des medizinischen Gerätes (D) ausführbar ist.

4. Verfahren nach einem der vorstehenden Ansprüche, bei dem der Identifikationsalgorithmus (22) als selbstlernendes System ausgebildet ist, indem der berechnete Austauschdatensatz (ad) und ein Bewertungsdatensatz (bd) für die jeweilige Fehlermeldung (fm) auf zugeordnete Weise in das Datenbanksystem (DB) eingepflegt wird.

5. Datenbanksystem (DB), welches einen Prozessor zu einer Ausführung des Verfahrens nach einem der Ansprüche 1 bis 4 umfasst, wobei die Fehlermustererzeugungseinheit (FME) zur Erzeugung der Fehlermuster (fmu) als Eingangsgrößen (e) aggregierte Testdaten eines Testbetriebs (TB) mit Fehlerfällen des medizinischen Gerätes (D) und zugeordneten Austauschdatensätzen einliest, um daraus ein Simulationsmodell mit Simulationsdaten zu erzeugen, das kontinuierlich mit weiteren Daten angereichert wird.

6. Datenbanksystem (DB) nach einem der vorstehenden auf das Datenbanksystem (DB) gerichteten Ansprüche, wobei das Simulationsmodell der Fehlermustererzeugungseinheit (FME) auch im Gerätebetrieb (GB) mit realen weiteren Daten ständig erweitert wird, so dass das Datenbanksystem (DB) als Eingangsgrößen (e) aggregierte Simulationsdaten eines Simulationsbetriebs des medizinischen Gerätes (D) mit Fehlerfällen einliest, um daraus Fehlermuster (fmu) zu erzeugen.

7. Datenbanksystem (DB) nach einem der vorstehenden auf das Datenbanksystem (DB) gerichteten Ansprüche, wobei das Datenbanksystem (DB) zentral für eine Vielzahl von medizinischen Geräten (D) ausgebildet ist.

8. Datenbanksystem (DB) nach einem der vorstehenden auf das Datenbanksystem (DB) gerichteten Ansprüche, wobei die Fehlermustererzeugungseinheit (FME) mit einer Regelbasis (RDB) interagiert, in der Regeln zum Erzeugen der Fehlermuster (fmu) abgelegt sind.

9. Datenbanksystem (DB) nach einem der vorstehenden auf das Datenbanksystem (DB) gerichteten Ansprüche, wobei die Fehlermustererzeugungseinheit (FME) zur Erzeugung von Fehlermustern (fmu) einen Bewertungsdatensatz (bd) berücksichtigt, der ein Maß für die Güte einer Fehlerbehebung repräsentiert und der bei historischen Bauteilersetzungen zu einer Fehlermeldung (fm) erfasst und gespeichert worden ist.

10. Berechnungseinheit (S) zum Anbieten eines Bauteilidentifikationsdienstes, der nach einem Verfahren nach den vorangehenden Verfahrensansprüchen betrieben wird und dazu mit dem Datenbanksystem (DB) interagiert und folgende Module umfasst:
- eine Eingangsschnittstelle (S1), die zum Einlesen von jeweils einer Fehlermeldung (fm) eines der medizinischen Geräte (D) bestimmt ist;
- einer Identifikationseinheit (S2), die zum Anwenden eines Identifikationsalgorithmus (22) zur Berechnung eines Austauschdatensatzes (ad) ausgebildet ist;
- einer Ausgabeschnittstelle (S3), die zur Ausgabe des berechneten Austauschdatensatzes (ad) bestimmt ist.

11. Medizinisches System (100) mit über ein Netzwerk (NW) verbundenen Einheiten, umfassend:
- Eine Mehrzahl von medizinischen Geräten (D), insbesondere von MR-Geräten, die jeweils eine Vielzahl von Bauteilen (B) umfassen;
- Eine Berechnungseinheit (S) nach dem unmittelbar vorangehenden Patentanspruch;
- Einem Datenbanksystem (DB) gemäß einem der vorangehenden auf das Datenbanksystem (DB) gerichteten Ansprüche.

12. Computerprogrammprodukt zur Identifikation von auszutauschenden Bauteilen eines medizinischen Gerätes (D), wobei das Computerprogrammprodukt auf einem Datenträger (MEM) gespeichert ist und Computerprogrammcode zur Ausführung des Verfahrens nach einem der vorangehenden Verfahrensansprüche auf einem Prozessor eines Computers umfasst.

## Claims

1. Method for the identification of structural parts (B) to be exchanged of a medical device (D) comprising in each case a plurality of structural parts, in particular an MR device, with the following method steps:
- reading-in (21) of an error message (fm) from the device (D) ;
- execution of an identification algorithm (22) for calculating and outputting an exchange data set (ad) identifying a structural part to be exchanged (B) for the read-in error message (fm), wherein the identification algorithm (22) with the read-in error message (fm) accesses a database system (DB) in which error patterns (fmu) with exchange data sets are stored which have been calculated from a simulation model in order to calculate as the result the exchange data set (ad) associated with the read-in error message (fm), wherein in the database system (DB) error patterns (fmu) are stored which are generated automatically by means of an error pattern generation unit (FME), wherein the error pattern generation unit (FME) is embodied to execute a machine-learning method based on a simulation model that enables the continuous learning and storage of error patterns (fmu) by the correlation of input variables (e), wherein in each case an error pattern (fmu) comprises an association between an error type or a combination of error types and an exchange data set (ad) indicating a structural part (B) to be exchanged and wherein the database system (DB) or the error pattern generation unit (FME) comprises a processor (P) to generate the error pattern (fmu) or interacts therewith, wherein the processor (P) is embodied for generating the error pattern (fmu) for applying Markov processes.

2. Method according to claim 1, in which the execution of the identification algorithm (22) is triggered by the reading-in (21) of the error message (fm).

3. Method according to one of the preceding claims in which the method can be carried out without operating data and immediately after the commissioning of the medical device (D).

4. Method according to one of the preceding claims, in which the identification algorithm (22) is embodied as a self-learning system in that the calculated exchange data set (ad) and an evaluation data set (bd) for the respective error message (fm) is entered into the database system (DB) in the associated manner.

5. Database system (DB), which comprises a processor for executing the method according to one of claims 1 to 4, wherein to generate the error patterns (fmu), the error pattern generation unit (FME) reads in test data of a test operation (TB) aggregated as input variables (e) with error scenarios of the medical device (D) and associated exchange data sets in order to generate therefrom a simulation model with simulation data that is continuously enriched with further data.

6. Database system (DB) according to one of the preceding claims directed at the database system (DB), wherein the simulation model of the error pattern generation unit (FME) is also continuously expanded with real further data during device operation (GB) so that the database system (DB) reads in, aggregated as input variables (e), simulation data of a simulation operation of the medical device (D) with error scenarios in order to generate error patterns (fmu) therefrom.

7. Database system (DB) according to one of the preceding claims directed at the database system (DB), wherein the database system (DB) is embodied centrally for a plurality of medical devices (D).

8. Database system (DB) according to one of the preceding claims directed at the database system (DB), wherein the error pattern generation unit (FME) interacts with a rule base (RDB) in which rules for generating the error patterns (fmu) are stored.

9. Database system (DB) according to one of the preceding claims directed at the database system (DB), wherein, to generate error patterns (fmu), the error pattern generation unit (FME) takes account of an evaluation data set (bd) representing a measure of the quality of an error correction and which has been acquired and stored during historical structural part replacements to form an error message (fm).

10. Calculation unit (S) for offering a structural part identification service which is operated in accordance with a method according to the preceding method claims and, to this end, interacts with the database system (DB) and comprises the following modules:
- an input interface (S1), which is intended in each case to read in an error message (fm) for one of the medical devices (D);
- an identification unit (S2), which is embodied to use an identification algorithm (22) to calculate an exchange data set (ad);
- an output interface (S3), which is intended to output the calculated exchange data set (ad).

11. Medical system (100) with units connected via a network (NW) comprising:
- a plurality of medical devices (D), in particular MR devices, which in each case comprise a plurality of structural parts (B);
- a calculation unit (S) according to the immediately preceding claim;
- a database system (DB) according to one of the preceding claims directed at the database system (DB).

12. Computer program product for the identification of structural parts to be exchanged of a medical device (D), wherein the computer program product is stored on a data medium (MEM) and comprises computer program code for carrying out the method according to one of the preceding method claims on a processor of a computer.

## Revendications

1. Procédé d'identification de composants (B) à remplacer d'un appareil (D) médical, qui comprend respectivement une pluralité de composants, notamment d'un appareil RM, ayant les stades de procédé suivants :
- lecture (21) d'un message (fm) de défaut de l'appareil (D) ;
- exécution d'un algorithme (22) d'identification pour calculer et sortir un ensemble (ad) de données de remplacement, identifiant un composant (B) à remplacer, du message (fm) de défaut lu, l'algorithme (22) d'identification accédant par le message (fm) de défaut lu à un système (DB) de base de données, dans lequel des modèles (fmu) de défaut sont mis en mémoire avec des ensembles de données de remplacement, qui ont été calculés à partir d'un modèle de simulation, pour calculer comme résultat l'ensemble (ad) de données de remplacement, qui doit être associé au message (fm) de défaut lu, dans lequel, dans le système (DB) de base de données, sont mis en mémoire des modèles (fmu) de défaut, qui sont produits automatiquement au moyen d'une unité (FME) de production de modèles de défaut, l'unité (FME) de production de modèles de défaut étant constituée pour réaliser un procédé d'apprentissage automatique sur la base d'un modèle de simulation, qui sert à l'apprentissage continu et à la mémorisation de modèles (fmu) de défaut par corrélation de grandeurs (e) d'entrée, dans lequel respectivement un modèle (fmu) de défaut comprend une association entre un type de défaut ou une combinaison de types de défaut et un ensemble (ad) de données de remplacement, qui indexe un composant (B) à remplacer, et dans lequel le système (DB) de base de données ou l'unité (FME) de production de modèles de défaut comprend, pour la production du modèle (fmu) de défaut, un processeur (P) ou est en relation d'échange de données avec celui-ci, le processeur (P) étant, pour la production du modèle (fmu) de défaut, constitué pour l'utilisation de processus de Markov.

2. Procédé suivant la revendication 1, dans lequel on déclenche l'exécution de l'algorithme (22) d'identification par la lecture (21) du message (fm) de défaut.

3. Procédé suivant l'une des revendications précédentes, dans lequel le procédé peut être effectué sans données de fonctionnement et directement après la mise en service de l'appareil (D) médical.

4. Procédé suivant l'une des revendications précédentes, dans lequel l'algorithme d'identification est constitué en système à auto-apprentissage par le fait que l'ensemble (ad) de données de remplacement calculées et un ensemble (bd) de données d'évaluation du message (fm) de défaut respectif sont insérés de façon associée dans le système (DB) de base de données.

5. Système (DB) de base de données, qui comprend un processeur pour une exécution du procédé suivant l'une des revendications 1 à 4, l'unité (FME) de production de modèles de défaut visant, pour la production du modèle (fmu) de défaut comme grandeur (e) d'entrée, des données de test agrégées d'un fonctionnement (TB) de test ayant des cas de défaut de l'appareil (D) médical et des ensembles de données de remplacement associés pour en produire un modèle de simulation ayant des données de simulation, qui est enrichi continuellement avec d'autres données.

6. Système (DB) de base de données suivant l'une des revendications précédentes visant le système (DB) de base de données, dans lequel le modèle de simulation de l'unité (FME) de production de modèles de défaut est étendu aussi constamment dans le fonctionnement (TB) de l'appareil par d'autres données réelles, de manière à ce que le système (DB) de base de données lise des données de simulation, agrégées en grandeur (e) d'entrée, d'un fonctionnement de simulation de l'appareil (D) médical avec des cas de défaut pour en produire un modèle (FME) de défaut.

7. Système (DB) de base de données suivant l'une des revendications précédentes visant le système (DB) de base de données, dans lequel le système (DB) de base de données est constitué de manière centralisée pour une pluralité d'appareils (D) médicaux.

8. Système (DB) de base de données suivant l'une des revendications précédentes visant le système (DB) de base de données, dans lequel l'unité (FME) de production de modèles de défaut interagit avec une base (RDB) de règle, dans laquelle sont mises des règles pour la production du modèle (fmu) de défaut.

9. Système (DB) de base de données suivant l'une des revendications précédentes visant le système (DB) de base de données, dans lequel l'unité (FME) de production de modèles de défaut prend en compte, pour la production de modèles (fmu) de défaut, un ensemble (bd) de données d'évaluation, qui représente une mesure de la qualité d'une suppression de défaut et qui a été relevé et mis en mémoire lors de remplacement historique de composants en un message (fm) de défaut.

10. Unité (S) de calcul pour offrir un service d'identification de composant pouvant fonctionner suivant un procédé selon les revendications de procédé précédentes pour interagir ainsi avec le système (DB) de base de données et qui comprend les modules suivants :
- une interface (S1) d'entrée, destinée à lire un message (fm) de défaut respectif de l'un des appareils (D) médicaux ;
- une unité (S2) d'identification, constituée pour l'utilisation d'un algorithme (22) d'identification pour calculer un ensemble (ad) de données de remplacement ;
- une interface (S3) de sortie, destinée à donner l'ensemble (ad) de données de remplacement qui a été calculée.

11. Système (100) médical ayant des unités reliées par un réseau (NW), comprenant :
- une pluralité d'appareils (D) médicaux, notamment d'appareils RM, qui comprennent chacun une pluralité de composants (B) ;
- une unité (S) de calcul suivant la revendication immédiatement précédente ;
- un système (DB) de base de données suivant l'une des revendications précédentes visant le système (DB) de base de données.

12. Produit de programme d'ordinateur pour l'identification de composants à remplacer d'un appareil (D) médical, le produit de programme d'ordinateur étant mis en mémoire sur un support (MEM) de données et comprenant un code de programme d'ordinateur pour l'exécution du procédé suivant l'une des revendications précédentes sur un processeur d'un ordinateur.
